# EUROPEAN PATENT APPLICATION

(11) **EP 4 516 208 A1**
(43) Date of publication of application: **05.03.2025**
(21) Application number: 23194105.5
(22) Date of filing: 29.08.2023
(51) Int. Cl.: A61B 5/00, A61B 5/053, A61B 5/0537

(54) **BIOMEDICAL SENSOR AND ASSOCIATED METHODS AND FABRICATION PROCESS**

(71) Applicant: Ordóñez, José Andrés Leal, 79114 Freiburg im Breisgau (DE)
(72) Inventor: Ordóñez, José Andrés Leal, 79114 Freiburg im Breisgau (DE)
(74) Representative: Mertzlufft-Paufler, Cornelius

(57) **Abstract**

An implantable biomedical sensor (1) is disclosed which features a novel electrofluidic design based on dedicated measurement chambers (2, 3) which are closed up by respective membranes (4, 5), which are permeable to water molecules and optionally also to typical ions which are found in the human interstitial fluid (ISF). Through this concept, the sensor (1) can perform electrical measurements in the respective chamber (2, 3) and thereby collect data which allow conclusions to be drawn about the amount of water and ions present in the ISF surrounding the sensor (1). In other words, such a sensor (1) enables electrical monitoring of an electrolyte status and/or a hydration status of the patient wearing the implanted sensor (1) in his tissue. (Fig.2)

## Description

The present disclosure relates to a biomedical sensor, which is designed for determining, in particular for (continuously) monitoring, an electrolyte status and/or a hydration status of a user/patient through respective electrical measurements. A specific use case of such a sensor and an efficient method for electrical determination of a water content and/or of an ionic content of an aqueous medium, respectively, in particular within human or within animal tissue, are also disclosed as well as a process for parallel wafer-level-fabrication of such biomedical sensors and a method for evaluating a sensor signal produced by such a sensor.

Today, a substantial amount of people over the age of 65 are hospitalized yearly for dehydration-related health deterioration. According to the World Population Prospects (United Nations, 2019), by the year 2050, the world will count 1 in 6 people over the age of 65. This demographic development as well as the climate change with elevated temperatures presents a major health and economic challenge to society.

No solution exists today to address the problem of accurately monitoring dehydration and electrolyte imbalances. Desirable would be a continuous, preventive and personalized healthcare monitoring solution that provides trustworthy access to the relevant health information. This is because enabling timely intervention and supporting health management is key to avoid the deterioration of health, in particular for elderly people.

Starting out from this background, it is one objective of the invention to provide a technical solution based on an implantable technology for continuous monitoring of hydration and electrolytes with clinically relevant accuracy intended for personalized prevention of health decay and for increased quality of life and autonomy of the patient.

In accordance with the present invention, a biomedical sensor is provided according to claim 1, which solves the aforementioned problem. The present invention thus proposes to employ an implantable biomedical sensor for accurately monitoring the electrolyte status and/or the hydration status of a patient.

In particular, the invention proposes a sensor as introduced at the beginning, which, in addition, is characterized in that the sensor comprises a first measurement chamber, which is closed by and in communication with the ambient via a porous first membrane (which may be preferably a stiff membrane), which is permeable to ions (such as Na⁺, Cl⁻, Mg²⁺ or Ca²⁺). In other words, the first measurement chamber can communicate via the first membrane with an ambient surrounding the sensor.

The sensor further preferably comprises a second measurement chamber, which is closed by and in communication with the ambient via a water-permeable second membrane which is impermeable to ions. The second membrane may be preferably elastic.

Furthermore, a first set of at least two measurement electrodes is arranged in the first measurement chamber and a second set of at least two measurement electrodes is arranged in the second measurement chamber (if present).

With such a sensor design, a continuous, reliable electrical measurement of the hydration status and of the electrolyte profile becomes possible with the proposition for unobtrusive monitoring of these parameters through connected digital systems, thereby facilitating visualisation, early intervention and prevention of unnecessary hospitalisations; moreover, associated comorbidities when deviating from ideal euhydration can be effectively avoided. By combining implantable technologies into a novel sensor, together with digital connectivity and intelligent algorithms, this sensor can measure the user's hydration status and electrolyte status and transmit corresponding measurement data to a digital monitoring platform (accordingly the sensor may feature necessary wireless communication means for such communications). Based on existing clinical evidence of healthy electrolyte and hydration levels, such a health platform can communicate tendencies and alarms in acute or chronic cases of dehydration or electrolyte excess or deficiencies in real-time. The sensor thus offers an efficient, reliable minimally invasive, and implantable solution to continuously monitor hydration and electrolyte status of a patient. This capability is enabled by the proposed choice of the measurement chamber design and the chosen membranes, as will be detailed below.

In other words, the sensor may be configured to perform a respective measurement of a respective electrical resistance of a respective liquid that is contained in the respective measurement chamber. Hence, the sensor can perform two independent electrical measurements, namely for determining the electrical resistance, in particular an impedance, of a first liquid contained in the first chamber (using a first set of electrodes arranged in the second chamber) and of a second liquid contained in the second chamber (using a second set of electrodes arranged in the second chamber).

We note here that typically the first membrane will also be permeable to water. In other words, both the first membrane and the second membrane may be permeable to water, with the possible distinction that the porous first membrane may be permeable to liquid water and ions, while the second membrane can be chosen such that it is only permeable to single water molecules (which will be the case for a PDMS membrane of 100 µm thickness, for example) but not for liquid water droplets.

The technical term "chamber closed by or delimited by a membrane" may be understood herein as meaning that the respective membrane forms a boundary that delimits (and thereby defines) a volume of the respective measurement chamber. Via the membrane, the respective measurement chamber can thus be in touch with the ambient tissue and body fluids, as soon as the sensor is implanted into a human (or animal) body (e.g., subcutaneously).

The term "impermeable to ions" may be interpreted technically as implying a sufficiently low penetration rate, such that the number of ions penetrating the membrane is so low that a measurement of a second electrical resistance using the second set of at least two measurement electrodes inside the second measurement chamber is not affected.

Based on an electrical measurement performed in the first measurement chamber, the sensor can (indirectly) measure an electrolyte concentration in the tissue surrounding the sensor, when the sensor is introduced or implanted into the human body (of the user/patient). Thereby, the current electrolyte status of the patient/user can be determined with the sensor. This is because the higher the electrolyte concentration in the ambient, the more ions will diffuse into the first measurement chamber, resulting in an electrically measurable decrease of the electrical resistance/ increase of the electrical conductivity of the first liquid contained in the first measurement chamber. We note at this point that the volume of the first liquid may be effectively invariable, in particular when using a stiff first membrane.

Likewise, based on an electrical measurement performed in the second measurement chamber, the sensor can (indirectly) measure a water content in the (surrounding) tissue (more precisely of the interstitial fluid (ISF) comprised in said tissue), when the sensor is introduced or implanted in the human body. Thereby, the current electrolyte status of the patient/user can be determined with the sensor. This is because the higher the water content in the ambient (and thus the "hydration status"), the more water molecules will diffuse into the second measurement chamber via the second membrane, resulting in an increase in the volume of the second (aqueous) liquid contained in the second measurement chamber (note that the second membrane may expand, such that the volume of the second liquid is variable). As the second membrane is, however, impermeable to ions, and therefore the number of ions will not change in the second measurement chamber, an infusion of water molecules will result in a decrease of the ion concentration (measured in number of ions / mL volume of the second liquid) and thus a measurable increase of the electrical resistance/ decrease of the electrical conductivity of the second liquid contained in the second measurement chamber.

Depending on the application case, a simpler design of a biomedical sensor according to the inventive concept proposed herein could be a biomedical sensor according to claim 2, which also solves the afore-mentioned problem. This sensor already allows electrical determination of a hydration status of a patient. In particular the invention therefore proposes a sensor as introduced at the beginning, which, in addition, is characterized in that the sensor comprises a measurement chamber, which is closed by and in communication with the ambient via a, preferably elastic, water-permeable membrane which is impermeable to ions (such as Na⁺, Cl⁻, Mg²⁺ or Ca²⁺). Moreover, a set of at least two measurement electrodes is arranged in the chamber. It is understood here that the measurement chamber just described may be the second measurement chamber described with correspondence to claim 1 and that, accordingly, the mentioned set of at least two electrodes may be said second set and the mentioned membrane may be said second membrane.

The sensor(s) presented so far can be further elaborated and implemented in various ways, which are described in the subclaims and in the following:
For example, as mentioned the sensor is preferably designed for implantation into the human body. For this purpose, the sensor may feature a biocompatible (outer) encapsulation. In other words, the sensor may be designed to be introduced (permanently or temporarily, for example in case of designing the sensor as part of a probe) into the human body. For example, the sensor can be designed as a subcutaneous sensor and/or as part of a catheter designed for insertion into the human body. We note here that in the field of implant technology, implant durations above 30 days are typically already considered "permanently". In other words, even if a designed lifetime of a sensor is only 2 years, it can already be considered a permanent implant. By contrast, in the case of using the sensor as part of a catheter, the duration of implantation may be only a few minutes to a few hours.

For best benefiting from the sensor design, it is of advantage if the sensor is configured to measure a first electrical resistance (in particular a first electrical impedance, most preferably a first electrical impedance spectrum of said first liquid) with the first set of electrodes. Alternatively or additionally, it is likewise of advantage, if the sensor is configured to measure a second electrical resistance (in particular a second electrical impedance, most preferably a second electrical impedance spectrum of said second liquid) with the second set of electrodes. Such measurements can be automatically triggered by a control unit of the sensor.

An appropriate choice of materials for the membranes can be as follows: the first membrane can be made from a ceramic material, preferably from aluminum oxide (Al2O3). Using a ceramic membrane as the first membrane is of advantage, because this safeguards that the first membrane shows a very low water absorption and practically no mechanical stress due to swelling in water, as it is often observed for polymer membranes, in particular membranes made from polyurethane (PU). Avoiding mechanical stress is important for achieving a reliable long-term bonding of the first membrane (e.g., to a spacer forming the first measurement chamber) and for maintaining a constant/invariable volume of the first measurement chamber (the volume should remain as invariable as possible for an accurate measurement).

Moreover, the first membrane can be chosen such that it offers nanoscale pores, preferably with a maximum pore size below 1 pm, most preferably with a maximum pore size below 200 nm.

The first membrane can feature a thickness of at least 50 µm for providing sufficient stiffness. In such a case, it is most preferable, if the thickness of the first membrane is below 500 µm on the other hand, to enable a compact design.

The mentioned electrodes of the sensor can comprise platin (Pt), either in the form of Pt or Pt/Ir, and/or Iridium, in particular IrOx.

Most easily, the second membrane can be made from a polymer material, preferably from polydimethylsiloxane (PDMS).

Moreover, the second membrane can feature a thickness of at least 100 pm, and preferably of at least 200 µm. In such cases, it will be preferably if the thickness is also below 500 pm, for enabling a compact design.

As already mentioned, the first measurement chamber is preferably filled with a first liquid (which may be in the form of a gel, for example). In this case, it is preferable if said first liquid cannot diffuse through said first membrane out of the first measurement chamber. In other words, the first liquid will be trapped in the first chamber.

According to a specific embodiment, the first membrane is permeable to a class of ions, wherein said class comprises at least one of the following ions: K+, Na+, Cl-, Mg2+, Fe2+, Fe3+, Cu2+, Ca2+. Through such a design, the sensor can be rendered capable of specifically and electrically measuring concentrations of ions belonging to said class of ions within the first measurement chamber.

Moreover, the first membrane can be impermeable to a class of large-sized objects, wherein said second class comprises at least one of the following objects: blood cells, proteins, for example proteins with a minimum diameter of x nm, bacteria. The advantage of this choice and design of the first membrane is that biofouling due to the presence of such objects inside the first measurement chamber can be prevented. Moreover, a reliable long-term stable and selective (indirect) electrical measurement of the ion concentration of the interstitial fluid (ISF) surrounding the first measurement is achievable without interference from cells, proteins and other large-sized objects present in the ISF.

On the other hand, the second measurement chamber may be filled with a, preferably aqueous, second liquid containing a defined and constant amount/number of ions. Through this feature, a number/concentration of ions present in the second liquid will be well-defined and/or constant over time. Accordingly, an osmotic pressure (dropping) via the second membrane can be well-defined. Another consequence will be that the sensor will then be capable of specifically and electrically measuring the amount of water contained in the second measurement chamber at a certain point in time, in particular after water has diffused into or out of the second measurement chamber through the second membrane, as explained previously. As this diffusion depends on the water content of the surrounding ISF, i.e., the hydration status of the user/patient, this approach allows indirect but accurate (in particular without any significant temporal delay) electrical measurement of the hydration status.

One embodiment suggests that the sensor comprises a substrate and that the first and/or second set of electrodes comprise(s) at least two planar electrodes (respectively) deposited on said substrate. In such a design, it is preferable if the electrodes are electrically connected to respective electrically conductive vias formed in the electrically insulating substrate. This allows a very electrically reliable sensor design which is well-suited for implantation. Most preferably, said planar electrodes may be structured by laser machining, most preferably by laser ablation, or by screen printing or by photolithography. All these techniques allow sufficient resolution for accurately defining the necessary electrode geometries.

The substrate may be a ceramic substrate or a silicon substrate, for example. As mentioned, depending on the application, the structuring of the electrodes can be done by screen printing based on thick film technology, or through any photolithography method based on thin-film technology.

Another design, which can be combined with the previously mentioned features, proposes that the sensor comprises a substrate (in particular said substrate with vias mentioned before) and the first and second measurement chambers are, in particular each, defined by a spacer attached to said substrate. In other words, there may be one or at least two spacers. The respective spacer can define sidewalls of the respective chamber. In addition, the respective spacer can provide a (preferably planar) surface to which the first membrane and/or the second membrane is/are bonded.

The sensor may also comprise a controller configured to perform (i) a first electrical impedance measurement of said first liquid, using the first set of electrodes arranged inside the first measurement chamber and/or (ii) a second electrical impedance measurement of said second liquid using the second set of electrodes arranged inside the second measurement chamber. From these measurements, the controller may compute derived data indicating the current hydration status and/or electrolyte status. The controller can also be configured to transmit such data or measurement results, preferably wirelessly, to an external communication device (a computer, smartphone etc.). Using impedance measurement specifically allows more accurate evaluation of the hydration and/or electrolyte status, because a larger amount of measurement points will be available.

Of course, the sensor may then comprise the necessary hardware for performing such impedance measurements, such as AC current source with adjustable frequency. The sensor can thus comprise a current source configured to deliver an alternating current to said first and/or second set of electrodes, in particular such that an AC measurement of an electrical impedance can be performed by the sensor.

According to one preferred design, the sensor may comprise a controller (in particular said mentioned controller) configured to perform a variation, most preferably a continuous sweep, of a frequency of an alternating current applied to said first or second set of electrodes over a certain frequency range for performing impedance spectroscopy (EIS). In this case, it is preferable if the controller is configured to compute a real part and an imaginary part from such recorded impedance spectra.

The sensor may comprise a voltmeter configured to perform a voltammetry measurement with the first and/or second set of electrodes and/or an amperemeter configured to perform an amperometry measurement with the first and/or second set of electrodes and/or a potentiometer configured to perform a potentiometry measurement with the first and/or second set of electrodes.

The sensor can also comprise a controller (in particular said mentioned controller) configured to compute an ion concentration of a/the second liquid contained in the second measurement chamber and/or to compute a water content currently enclosed/comprised in the second measurement chamber (based on an assumed constant amount of ions contained in the second measurement chamber). The (indirect) electrical measurement of the water content in the second measurement chamber (a measured low ion concentration will be synonymous to a high water content, due to the constant amount of ions contained in the second chamber) can be valuable for determining the hydration status of the patient/user: the more water contained in the interstitial fluid (ISF) and the lower the ion content of the ISF, the higher the likelihood of water diffusing from the ISF into the second measurement chamber, due to the osmotic pressure dropping over the second membrane. Accordingly, a determined high water content/low ion concentration in the second measurement chamber will be indicative of a high hydration status of the patient/user.

The first membrane can be so stiff that a liquid volume contained in the first measurement chamber, in particular a volume of said first liquid filling up the first chamber completely, is invariable within less than 1%, in particular even though ions and/or water can freely diffuse through said first membrane.

By contrast, a liquid volume contained in the second measurement chamber and delimited by the second membrane, in particular a volume of said second liquid filling up the second chamber completely, can vary, in particular as a result of water diffusing through said second membrane.

In a preferred design, the first and/or the second set of electrodes comprises (respectively) at least four electrodes. In such a case, the sensor may be configured for performing a 4-electrode-resistance measurement with the first and/or the second set of electrodes.

For achieving a compact design suitable for implantation, the electrodes of the first and second set may be micro-electrodes with sub-mm dimensions. Moreover, the first measurement chamber and/or the second measurement chamber can each comprise a volume of less than 1×1mm² × 0.3 mm = 0.3 µL to provide one possible example.

In a more advanced design, the sensor features at least two separate first measurement chambers, wherein one of the two first chambers is closed by and separated from the ambient by a porous type A membrane, which is impermeable to a (in particular second) class of large-sized ions. Preferably, said second class comprises at least one of the following ions: Ca2+, Mg²⁺. The other of the two first chambers may be closed by and separated from the ambient by a porous type B membrane, which is permeable to said, in particular second, class of large-sized ions. Thereby, an ion concentration of a/the first class of ions can be electrically and specifically measured with the first chamber (2a) covered by the type A membrane and/or an ion concentration of the second class of ions can be electrically and specifically measured with the first chamber covered by the type B membrane. Accordingly, such a sensor design allows to collect even more information on the current electrolyte status and a specific measurement of certain classes of ions.

The sensor can also feature a reference measurement chamber which is fully closed such that no ions and no water can penetrate from the ambient into the reference measurement chamber and wherein a third set of at least two measurement electrodes is arranged in the reference measurement chamber for performing electrical reference measurements. Preferably, a third liquid with a defined electrical conductivity, most preferably in the form of a hydrogel, can be contained in the reference measurement chamber. The advantage of such a reference measurement chamber is that the sensor can be configured to perform electrical reference measurements using the third set of electrodes. Based on the results of such a reference measurement, temperature drifts and other variations can be detected and compensated (e.g. by a controller of the sensor).

For solving the aforementioned problem, the approach of the invention can also result in a specific use of a sensor as proposed herein (which may have features as claimed and/or as described herein). This use is characterized in that the sensor is used for monitoring the hydration status and/or the electrolyte status of a user/patient. Such monitoring may be performed continuously and/or repeatedly (e.g., in preset time intervals). In such use cases, the sensor may be implanted in or introduced into the user's body while/before the monitoring is performed. Moreover, the sensor can be configured such that it performs the electrical measurements autonomously.

As outlined at the beginning, we also propose a method for electrical determination of a water content and/or of an ionic content of an aqueous medium, respectively, in particular within human or animal tissue, using a biomedical sensor (which may have features as claimed or described herein). The sensor should be in touch with the medium, in particular with said tissue. The method is distinguished in that an ionic concentration within a first measurement chamber of the sensor containing a first liquid is electrically measured by the sensor with a first set of electrodes in a first measurement for determining the ionic content in the medium / tissue. This may be done, in particular, by electrically measuring an electrical resistance and/or an electrical impedance of said first liquid, as described before. It is understood that during such measurements, ions may freely diffuse through the described first membrane into and out of said first measurement chamber.

In addition or as an alternative, following the above approach, a water content or an ionic concentration within a second measurement chamber of the sensor containing a second liquid can be electrically measured by the sensor with a second set of electrodes in a second measurement (preferably independent of the first measurement) for determining the water content in the medium / tissue. This may be done, in particular, by electrically measuring an electrical resistance and/or an electrical impedance of said first liquid.

As mentioned, ions and/or water may diffuse through a porous first membrane into the first measurement chamber during said first measurement and/or water molecules may diffuse through a second membrane into the second measurement chamber during said second measurement. The respective chamber can shielded / separated from the ambient, in particular from neighboring human or animal cells, by the respective membrane.

In this method, the first measurement and/or the second measurement may be performed using an alternating current, respectively, which is provided by a current source of the sensor: For example, the first measurement and/or the second measurement may thus include a variation, in particular a sweep, of a frequency of the respective alternating current, such that an impedance spectroscopy is performed, respectively.

The method can also be designed and applied to allow autonomous monitoring of a hydration state and an electrolyte state of a patient. For example, the sensor, after having been introduced or implanted into the body of the patient, may transmit data of/related to said first and/or second measurement to an extracorporeal monitoring device (a tablet, smart phone or the like), which outputs a current (i.e. measured at this particular point in time) measurement value, in particular a measurement trend, related to the hydration status and the electrolyte status of the patient, based on the data transmitted by the sensor. Preferably, the monitoring device can trigger the sensor, for example by transmitting an instruction to the sensor to perform a new first and second measurement. This instruction can of course depend on the data previously transmitted by the sensor to the monitoring device, i.e., the monitoring device can autonomously decide whether to trigger further measurements or not.

Finally, we also propose a specific process for parallel wafer-level fabrication of biomedical sensors (which have features as claimed and/or described herein), wherein each fabricated sensor can be designed or later configured as an implantable sensor. The process comprises the following steps:
- formation, preferably laser machining, of electrodes deposited on a wafer forming a common substrate for the sensors; In other words the sensor may share this substrate and may be later separated from each other by singulation.
- bonding of a spacer-wafer onto the substrate or pick-and-place assembly of spacers onto the substrate, the spacer(s) forming sidewalls of respective measurement chambers of the sensor;
- dispensing of a first liquid into a plurality of the first measurement chambers formed on the substrate;
- formation of a second liquid containing a defined concentration of ions in each of a plurality of second measurement chambers formed on the same substrate by dispensing a liquid, in particular said second liquid, into each of the second measurement chambers; We note that this formation could be achieved by dispensing of water only, in case a salt crystal is already present in each chamber, e.g. deposited by a pick-and-place machine.
- bonding of at least one first membrane (preferably bonding of a multitude of first membranes). Such bonding may be done using a pick-and-place robot, for example. The bonding can be done to the spacer-wafer or to said mentioned (multiple) spacers;
- bonding of at least one second membrane (preferably of a multitude of second membranes), preferably using a pick-and-place robot, to the spacer-wafer or to the spacer;
- singulation of the resulting wafer-level-stack comprising said substrate into individual dies; each die corresponds to one of said sensors.

The formation of the second liquid may be achieved by first depositing an ionic substance, for example a certain amount of an ionic salt, in each measurement chamber followed by dispensing of a liquid into the respective second measurement chamber. As an alternative, the final second liquid may be pre-fabricated and directly dispensed into the respective second measurement chamber.

The process may further include a step of encapsulating of the dies with a bio-compatible soft encapsulation material for forming the individual biomedical sensors. Of course, said first membranes and said second membranes can each be left at least partially free from said encapsulation material. Thereby, a transfer of ions and/or of water molecules through the respective membrane will remain possible.

The fabricated sensors can each show a planar design. Moreover, the sensors can be free of cables as all necessary electrical wiring can be integrated in or on the common substrate, in particular based on electrically conductive vias which are formed in the electrically insulating substrate. Such vias can connect the measurement electrodes on the upper side of the substrate with a current source and voltmeter arranged (in particular as separate electronic components) on the lower side of the substrate.

In detail, the singulated dies can each be bonded to a respective lower microelectronic package, the package comprising a respective microelectronic circuit that is electrically connected to the electrodes formed on the top side of the substrate (in the respective chamber). This connection may be formed through electrically conductive vias which are formed in the electrically insulating substrate.

Preferably the microelectronic circuit may implement an electronic controller of the sensor, with the controller configured to perform first and second electrical measurements in the chambers of the sensor to which the circuit belongs.

Finally, in the context of the biomedical sensors presented herein, we also propose a method for evaluating a sensor signal delivered by such a biomedical sensor (which may be fabricated as described above and/or have features as claimed and/or described herein). In this method, an alternating current of variable frequency is employed by the sensor for measuring an electrical impedance spectrum of a liquid contained in a measurement chamber of said sensor. In particular, said chamber may be closed by and separated from the ambient by a water-permeable membrane, as described before. The impedance spectrum (in particular a real and an imaginary part of a measured impedance curve) may then be evaluated using a method of machine learning. This may be realized, in particular, by employing an artificial intelligence (AI) that performs the evaluation, which can be implemented in software and/or hardware.

Based on such an evaluation, ion concentrations of specific ions can be computed. For example, the evaluation may be executed by a controller integrated in the sensor, in particular such that the sensor transmits data relating to the computed specific ion concentrations. Alternatively, the evaluation can also be executed by an external controller belonging to an extracorporeal monitoring device; in this case, said AI may be implemented by a software running on the monitoring device. The monitoring device may receive measurement data from said sensor, preferably wirelessly.

Preferred examples of the present invention shall now be described in more detail, although the present invention is not limited to these examples: for those skilled in the art, it is obvious that further examples of the present invention may be obtained by combining features of one or more of the patent claims with each other and/or with one or more features of an example as described or illustrated herein.

With reference to the accompanying drawings, where features with corresponding technical function are referenced with same numerals even when these features differ in shape or design:
- Fig. 1: is a top view onto a biomedical sensor according to the invention,
- Fig. 2: shows a schematic cross-sectional view on the sensor of Figure 1, and
- Fig. 3: illustrates how the sensor of Figures 1 and 2 can communicate with an extracorporeal monitoring device, thus forming a digital biomedical monitoring platform.

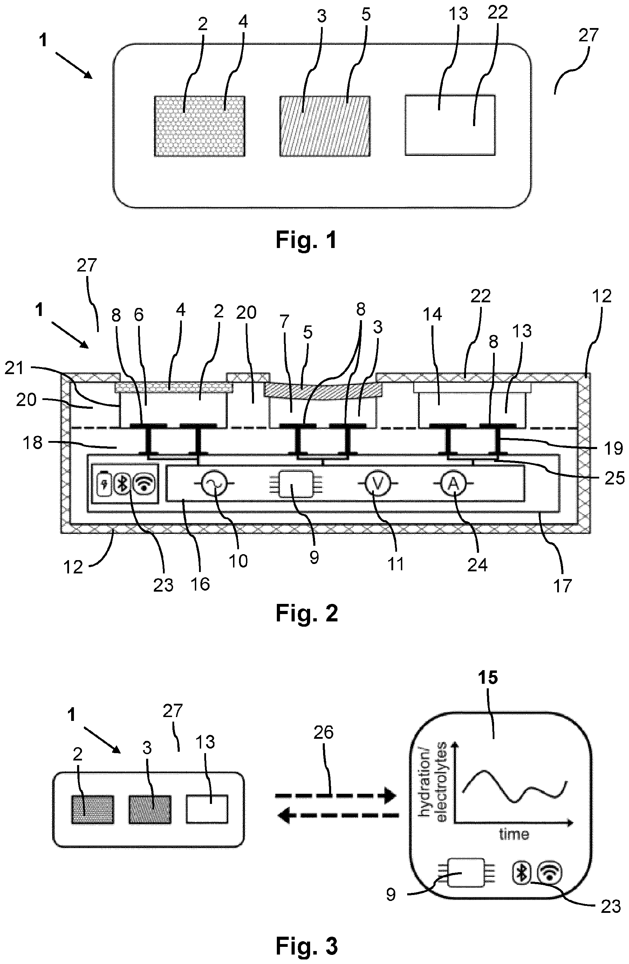

Figure 1 shows a possible embodiment of a sensor 1 according to the invention, which features three separate measurement chambers 2, 3 and 13: As can be seen more accurately in the cross-sectional view of Figure 2, the first measurement chamber 2 is closed by a ceramic first membrane 4 with nanometer-sized pores. When the sensor 1 is implanted in human tissue subcutaneously, ions and water molecules present in the interstitial fluid (ISF) in close vicinity to the sensor 1 can freely diffuse from the ambient 27 through said first membrane 4 into a first liquid 6, which is enclosed in the first measurement chamber 2 by the first membrane 4. As the first membrane 4 is stiff, the volume of the liquid 6 contained in the first measurement chamber 2 cannot change over time. This is also because the first liquid 6 is chosen such that it cannot diffuse through the first membrane 4 out of the first chamber 2. Accordingly, the concentration of ions present inside the first liquid 6 will quickly adapt to the corresponding ion concentration that is present in the surrounding ISF at a given point in time. This is because in case the ion concentration outside the first chamber 2 is higher than inside this chamber 2, ions will tend to diffuse into the first liquid 6 through the first membrane 4, thereby leveling the difference in ion concentrations.

As can be seen in Figure 2, the design of the second measurement chamber 3 is similar to that of the first measurement chamber 2, however the second liquid 7 contained in the chamber 2 (which may be different from the first liquid 6) is closed by a second deformable polymer membrane 5, which is impermeable to ions but permeable to single water molecules. The second aqueous liquid 7 is chosen such that its ion concentration is well known. If the amount of water in the ISF increases due to a high hydration status, more water molecules will penetrate the second membrane 5 and as this membrane 5 is flexible, the volume of the second liquid 7 will increase, due to the water intake. As a further result, the electrical conductivity of the second liquid 7 will drop, because the intake of water results in an effective decrease of the ion concentration inside the second measurement chamber 3 (note that all ions present in the second liquid 7 are trapped in the second chamber 3), because the number of ions inside this chamber 3 will not change / will be constant. By measuring the electrical conductivity and/or electrical resistance (which of course is technically equivalent) of the second liquid 7 in regular time intervals, the hydration status in the surrounding ambient 27 can be indirectly determined / monitored by the sensor 1.

Note that a simpler design of the sensor 1 could employ only such a second measurement chamber 3 but no first measurement chamber 2. This would already be sufficient for monitoring of a hydration status.

Figure 2 shows, that the sensor 1 further comprises an internal non-conductive substrate 18, which forms the bottom of each of the three chambers 2, 3, and 13. A spacer 20 is bonded on the upper side of the substrate 18, thereby forming respective sidewalls 21 of each chamber 2/3/13. On the upper side of the substrate 18, on the bottom of each of the chambers 2/3/13, respective measurement electrodes 8 are deposited, which are electrically connected by electrical vias 19 to a complex microelectronic package 17, which also forms part of the sensor 1. All of these components are encapsulated in a biocompatible encapsulation material 12 forming the outer surface of the sensor 1. Note that the membranes 4 and 5 of the first and second measurement chambers 2, 3 are left partially open, while the third measurement chamber 13, which serves as a reference measurement chamber, is fully closed by a cover 22, which is impermeable to both water and ions. In other words, there is no exchange between the ambient 27 and the third liquid 14 contained in said reference measurement chamber 13. We note that the sensor design shown in Figure 2 could be fabricated with the wafer-level-process previously explained; in this case the size of the sensor 1 shown schematically in Figure 2 will roughly equal the size of die cut out of the carrier wafer 18 / main substrate 18 employed in the process.

The microelectronic package 17 (which may be soldered to the vias 19 prior to or after singulation of the substrate 18 into single dies) comprises a controller 9, which operates a current source 10, a voltmeter 11 and an amperemeter 24 to perform various electrical measurements using the respective set of electrodes 8 present in each chamber 2, 3, 13. Note that these components 9,10,11,24 are all electrically connected to each other by electrical interconnects of the package 17. Through the vias 21, the controller can also electrically address the electrodes 8.

For example, the controller 9 can be programmed such that it electrically measures complex electrical impedance spectra of each liquid 6,7,14, respectively, using said components 9,10,11,24 and the electrodes 8. By considering the data resulting from electrical measurements performed in the reference measurement chamber 13, the controller 9 can compensate for temperature drifts. Note that the composition of the third liquid 14 and its volume do not change over time.

As illustrated in Figure 3, the sensor 1 can transmit such measured data and/or data derived from such measurements by the controller 9 using a wireless communication module 23, which also forms part of said microelectronic package 17. The data are sent from the sensor 1 to a monitoring device 15, for example a tablet, which is located outside of the patient's body. This monitoring device 15 can then further evaluate the transmitted data, in particular based on an algorithm that implements an artificial intelligence, and can for example compute current trends of the electrolyte status and the hydration status of the patient. Such trends can then be visualized on a screen of the device 15 and can instruct the patient to an adequate behavior, e.g., drinking more water.

In summary, an implantable biomedical sensor 1 is disclosed which features a novel electrofluidic design based on dedicated measurement chambers 2, 3 which are sealed/closed up by respective membranes 4, 5, which are permeable to water molecules and, in the case of the first membrane 4, also to typical ions which are found in the human interstitial fluid (ISF). Through this concept, the sensor 1 can perform electrical measurements in the respective chamber 2, 3 and thereby collect data which allow conclusions to be drawn about the amount of water and ions present in the ISF surrounding the sensor 1. In other words, such a sensor 1 enables electrical monitoring of an electrolyte status and/or a hydration status of the patient wearing the implanted sensor 1 in his tissue.

### List of reference numerals

- 1: sensor (to be implanted into the human body, in particular subcutaneously)
- 2: first measurement chamber
- 3: second measurement chamber (permeable to ions below a specific size)
- 4: first membrane (porous, and preferably stiff)
- 5: second membrane (elastic, water-permeable)
- 6: first liquid
- 7: second liquid
- 8: electrodes (for measuring electrical resistance and/or impedance)
- 9: controller (can control current source and voltmeter comprised in 1)
- 10: current source (preferably AC current source)
- 11: voltmeter
- 12: encapsulation material
- 13: reference measurement chamber
- 14: third liquid
- 15: monitoring device
- 16: microelectronic circuit
- 17: microelectronic package
- 18: substrate
- 19: vias (electrically conductive, formed in 18)
- 20: spacer
- 21: sidewall (of 2, 3; formed by 20)
- 22: cover (impermeable to water and ions)
- 23: wireless communication module
- 24: amperemeter
- 25: electrical interconnects (formed in 17)
- 26: wireless communication
- 27: ambient (tissue and ISF)

## Claims

1. **Biomedical sensor** (1) for determining, in particular for monitoring, an electrolyte status and a hydration status of a user or patient through respective electrical measurements, the sensor (1) comprising
- a first measurement chamber (2), which is closed by and in communication with the ambient (27) via a, preferably stiff, porous first membrane (4) which is permeable to ions, and
- a second measurement chamber (3), which is closed by and in communication with the ambient (27) via a, preferably elastic, water-permeable second membrane (5) which is impermeable to ions,
- wherein a first set of at least two measurement electrodes (6) is arranged in the first measurement chamber (2) and a second set of at least two measurement electrodes (6) is arranged in the second measurement chamber (3).

2. **Biomedical sensor** (1), in particular according to claim 1, for determining, in particular for monitoring, a hydration state of a user or patient through an electrical measurement, the sensor (1) comprising
- a measurement chamber (3), which is closed by and in communication with the ambient via a, preferably elastic, water-permeable membrane (5) which is impermeable to ions,
- wherein a set of at least two measurement electrodes (6) is arranged in the measurement chamber (3),
- in particular wherein the measurement chamber (3) is the second measurement chamber (3) and the set of at least two electrodes (8) is said second set and the membrane (5) is said second membrane (5).

3. Sensor (1) according to claim 1 or 2, wherein the sensor (1) is designed for implantation into the human body, in particular wherein the sensor (1) features a biocompatible encapsulation (12) for this purpose, or
- wherein the sensor (1) is designed to be introduced, permanently or temporarily, into the human body, in particular as a subcutaneous sensor (1) and/or as part of a catheter designed for insertion into the human body.

4. Sensor (1) according to one of the preceding claims,
- wherein the sensor (1) is configured to measure a first electrical resistance, in particular a first electrical impedance, most preferably a first electrical impedance spectrum, with the first set of electrodes (8) and/or
- wherein the sensor (1) is configured to measure a second electrical resistance, in particular a second electrical impedance most preferably a second electrical impedance spectrum, with the second set of electrodes (8).

5. Sensor (1) according to one of the preceding claims,
- wherein the first membrane (4) is made from a ceramic material, preferably from aluminum oxide (Al2O3) and/or
- wherein the first membrane (4) offers nanoscale pores, preferably with a maximum pore size below 1 pm, most preferably with a maximum pore size below 200 nm, and/or
- wherein the first membrane (4) features a thickness of at least 50 µm for providing sufficient stiffness, most preferably and wherein the thickness of the first membrane (4) is below 500 µm and/or
- wherein said electrodes (8) comprise platin (Pt), either in the form of Pt or Pt/Ir, and/or Iridium, in particular IrOx.

6. Sensor (1) according to one of the preceding claims,
- wherein the second membrane (5) is made from a polymer material, preferably from polydimethylsiloxane (PDMS), and/or
- wherein the second membrane (5) features a thickness of at least 100 pm, preferably of at least 200 pm, most preferably and below 500 µm.

7. Sensor (1) according to one of the preceding claims,
- wherein the first measurement chamber (2) is filled with a first liquid (7), preferably wherein said first liquid cannot diffuse through said first membrane (4) out of the first measurement chamber (2).

8. Sensor (1) according to one of the preceding claims,
- wherein the first membrane (4) is permeable to a class of ions, wherein said class comprises at least one of the following ions: K+, Na⁺, Cl⁻, Mg²⁺, Fe²⁺, Fe³⁺, Cu²⁺, Ca²⁺ and/or
- wherein the first membrane (4) is impermeable to a class of large-sized objects, wherein said second class comprises at least one of the following objects: blood cells, proteins with a minimum diameter of x nm, bacteria;
- in particular such that the sensor (1) is capable of specifically and electrically measuring concentrations of ions belonging to said class of ions within the first measurement chamber (2).

9. Sensor (1) according to one of the preceding claims,
- wherein the second measurement chamber (3) is filled with a, preferably aqueous, second liquid (7) containing a defined and constant amount of ions,
- in particular such that an amount of ions present in the second liquid (7) is well-defined and/or constant over time and/or
- such that an osmotic pressure via the second membrane (5) is well-defined and/or
- such that the sensor (1) is capable of specifically and electrically measuring the amount of water contained in the second measurement chamber (3) at a certain point in time, in particular after water has diffused into or out of the second measurement chamber (3) through the second membrane (5).

10. Sensor (1) according to one of the preceding claims,
wherein the sensor (1) comprises a substrate (18) and the first and/or second set of electrodes (8) comprise(s) at least two planar electrodes deposited on said substrate (18),
- preferably wherein the electrodes (8) are electrically connected to respective electrically conductive vias (19) formed in the electrically insulating substrate (18),
- most preferably wherein said planar electrodes (8) have been structured by laser machining, most preferably by laser ablation, or by screen printing or by photolithography.

11. Sensor (1) according to one of the preceding claims,
wherein the sensor (1) comprises a substrate (18) and the first and second measurement chambers (2, 3) are, in particular each, defined by a spacer (20) attached to said substrate (18), in particular wherein the respective spacer (20) defines sidewalls (21) of the respective chamber (2, 3) and/or
- the first membrane (4) and/or the second membrane (5) is bonded to said spacer (20).

12. Sensor (1) according to one of the claims 7 to 9, wherein the sensor (1) comprises a controller (9) configured to perform
- a first electrical impedance measurement of said first liquid (6), using the first set of electrodes (8) arranged inside the first measurement chamber (2) and/or
- a second electrical impedance measurement of said second liquid (7) using the second set of electrodes (8) arranged inside the second measurement chamber (3).

13. Sensor (1) according to the previous claim, wherein the sensor (1) comprises a current source (10) configured to deliver an alternating current to said first and/or second set of electrodes (8), in particular such that an AC measurement of an electrical impedance can be performed by the sensor (1).

14. Sensor (1) according to one of the preceding claims,
wherein the sensor (1) comprises a controller (9), in particular said mentioned controller (9), configured to perform a variation, most preferably a continuous sweep, of a frequency of an alternating current applied to said first or second set of electrodes (8) over a certain frequency range for performing impedance spectroscopy (EIS),
- preferably wherein the controller (9) is configured to compute a real part and an imaginary part from a recorded impedance spectrum.

15. Sensor (1) according to the one of the preceding claims,
- wherein the sensor (1) comprises a voltmeter configured to perform a voltammetry measurement with the first and/or second set of electrodes (8) and/or
- wherein the sensor (1) comprises an amperemeter (24) configured to perform an amperometry measurement with the first and/or second set of electrodes (8) and/or
- wherein the sensor (1) comprises a potentiometer configured to perform a potentiometry measurement with the first and/or second set of electrodes (8).

16. Sensor (1) according to one of the preceding claims,
wherein the sensor (1) comprises a controller (9), in particular said mentioned controller (9), configured to
- compute an ion concentration of a second liquid (7) contained in the second measurement chamber (3) and/or
- a water content currently enclosed/comprised in the second measurement chamber (3), based on an assumed constant amount of ions contained in the second measurement chamber (3).

17. Sensor (1) according to one of the preceding claims,
- wherein the first membrane (4) is so stiff that a liquid volume contained in the first measurement chamber (2), in particular a volume of said first liquid (6) filling up the first chamber (2), is invariable within less than 1%, in particular even though ions and/or water can freely diffuse through said first membrane (4), and/or
- wherein a liquid volume contained in the second measurement chamber (3) and delimited by the second membrane (5), in particular a volume of said second liquid (7) filling up the second chamber (3), can vary, in particular as a result of water diffusing through said second membrane (5).

18. Sensor (1) according to one of the preceding claims,
- wherein the first and/or the second set of electrodes (8) comprises at least four electrodes (8) and the sensor (1) is configured for performing a 4-electrode-resistance measurement with the first and/or the second set of electrodes (8).

19. Sensor (1) according to one of the preceding claims,
- wherein the electrodes (8) of the first and second set are micro-electrodes having sub-mm dimensions and/or
- wherein the first measurement chamber (2) and/or the second measurement chamber (3) each comprise a volume of less than 1×1mm² × 0.3 mm = 0.3 µL.

20. Sensor (1) according to one of the preceding claims,
- wherein the sensor features at least two separate first measurement chambers (2a, 2b),
- wherein one of the two first chambers (2a) is closed by and separated from the ambient by a porous type A membrane (4), which is impermeable to a, in particular second, class of large-sized ions, preferably wherein said second class comprises at least one of the following ions: Ca²⁺ or Mg²⁺;
- wherein the other of the two first chambers (2a) is closed by and separated from the ambient by a porous type B membrane (4), which is permeable to said, in particular second, class of large-sized ions,
- in particular such that an ion concentration of a/the first class of ions can be electrically and specifically measured with the first chamber (2a) covered by the type A membrane (4) and/or
- wherein an ion concentration of the second class of ions can be electrically and specifically measured with the first chamber (2a) covered by the type B membrane (4).

21. Sensor (1) according to one of the preceding claims,
- wherein the sensor (1) features a reference measurement chamber (13) which is fully closed such that no ions and no water can penetrate from the ambient into the reference measurement chamber (13) and wherein a third set of at least two measurement electrodes (6) is arranged in the reference measurement chamber (13) for performing electrical reference measurements,
- preferably wherein a third liquid (14) with a defined electrical conductivity, most preferably in the form of a hydrogel, is contained in the reference measurement chamber (13).

22. **Use of a sensor (1)** according to one of the preceding claims for, preferably continuously and/or repeatedly, monitoring
- the hydration status and
- the electrolyte status
of a user,
- wherein the sensor (1) is implanted in or introduced into the user's body while the monitoring is performed, - preferably wherein the sensor (1) performs the electrical measurements autonomously.

23. **Method for electrical determination of a water content and/or of an ionic content of an aqueous medium,** respectively, in particular within human or animal tissue,
- using a biomedical sensor (1), in particular according to one of the preceding claims 1 to 21, which is in touch with the medium, in particular said tissue,
- wherein an ionic concentration within a first measurement chamber (2) of the sensor (1) containing a first liquid (6) is electrically measured by the sensor (1) with a first set of electrodes (8) in a first measurement, in particular by electrically measuring an electrical resistance and/or an electrical impedance of said first liquid (6), for determining the ionic content in the medium / tissue
*and*/*or*
- wherein a water content or an ionic concentration within a second measurement chamber (3) of the sensor (1) containing a second liquid (7) is electrically measured by the sensor (1) with a second set of electrodes (8) in a second measurement, preferably independent of the first measurement, in particular by electrically measuring an electrical resistance and/or an electrical impedance of said first liquid (6), for determining the water content in the medium / tissue,
- in particular wherein ions and/or water diffuse(s) through a porous first membrane (4) into the first measurement chamber (2) during said first measurement and/or wherein water molecules diffuse through a second membrane (5) into the second measurement chamber (3) during said second measurement,
- most preferably wherein the respective chamber (2, 3) is shielded from the ambient, in particular from neighboring cells, by the respective membrane (4, 5).

24. Method according to the previous claim, wherein the first measurement and/or the second measurement are performed using an alternating current, respectively, which is provided by a current source of the sensor (1),
- in particular wherein the first measurement and/or the second measurement includes a variation, in particular a sweep, of a frequency of the respective alternating current, such that an impedance spectroscopy (EIS) is performed, respectively.

25. Method according to one of the preceding claims, in particular applied for achieving autonomous monitoring of a hydration state and a electrolyte state of a patient,
- wherein the sensor (1) has been introduced or implanted into the body of the patient and
- the sensor (1) transmits data of said first and/or second measurement to an extracorporeal monitoring device (15), which outputs a current measurement value, in particular a measurement trend, related to the hydration status and the electrolyte status of the patient, based on the data transmitted by the sensor (1),
- preferably wherein the monitoring device (15) triggers the sensor (1), in particular by transmitting an instruction to the sensor (1), to perform a new first and second measurement.

26. **Process for parallel wafer-level-fabrication of a multitude of biomedical sensors (1),** in particular wherein each sensor (1) is designed as an implantable sensor and/or according to one of the preceding claims 1 to 21, the process comprising the following steps:
- formation, preferably laser machining, of electrodes (8) deposited on a wafer forming a common substrate (18) for the sensors (1);
- bonding of a spacer-wafer onto the substrate (18) or pick-and-place assembly of spacers onto the substrate (18), the spacer(s) forming sidewalls of respective measurement chambers (2,3) of the sensors (1);
- dispensing of a first liquid (6) into a plurality of the first measurement chambers (2) formed on the substrate (18) ;
- formation of a second liquid (7) containing a defined concentration of ions in each of a plurality of second measurement chambers (3) formed on the same substrate (18) by dispensing a liquid, in particular said second liquid (7), into each of the second measurement chambers (3);
- bonding of at least one, preferably bonding of a multitude of, first membrane(s) (4), preferably using a pick-and-place robot, to the spacer-wafer or to the spacers;
- bonding of at least one, preferably of a multitude of, second membrane(s) (5), preferably using a pick-and-place robot, to the spacer-wafer or to the spacer;
- singulation of the resulting wafer-level-stack comprising said substrate (18) into individual dies, which each die corresponds to one of said sensors (1);

27. Process (1) according to the previous claim, wherein the process further includes a step of
- encapsulating of the dies with a bio-compatible soft encapsulation material (12) for forming the individual biomedical sensors (1) but leaving each of said first membranes (4) and said second membranes (5) at least partially free from said encapsulation material (12).

28. Process (1) according to one of the two preceding claims, wherein the fabricated sensors (1) each
- show a planar design and/or
- are free of cables as all necessary electrical wiring is integrated in or on the common substrate (18), in particular based on electrically conductive vias which are formed in the electrically insulating substrate (18).

29. Process (1) according to one of the claims 26 to 28,
wherein the singulated dies (1) are each bonded to a respective lower microelectronic package (17) comprising a respective microelectronic circuit (16) that is electrically connected to the electrodes (8) formed on the top side of the substrate (18) in the respective chamber (2, 3) through electrically conductive vias (18) which are formed in the electrically insulating substrate (18),
- preferably wherein the microelectronic circuit (16) implements a controller (9) of the sensor (1) configured to perform first and second electrical measurements in the chambers (2, 3) of the sensor (1) to which the circuit (16) belongs.

30. **Method for evaluating a sensor signal delivered by a biomedical sensor (1**), in particular a sensor (1) according to one of the claims 1 to 21 or fabricated with a process according to claims 26 to 29,
- wherein an alternating current of variable frequency is employed by the sensor (1) for measuring an electrical impedance spectrum of a liquid (6,7) contained in a measurement chamber (2,3) of said sensor (1),
- in particular wherein said chamber (2, 3) is closed by and separated from the ambient by a water-permeable membrane (4,5),
- wherein the impedance spectrum, in particular a real and an imaginary part of a measured impedance curve, is/are evaluated using a method of machine learning, in particular by employing an artificial intelligence, and
- wherein based on said evaluation, ion concentrations of specific ions are computed,
- in particular wherein said evaluation is executed by a controller (9) integrated in the sensor (1), in particular such that the sensor (1) transmits data relating to the computed specific ion concentrations or
- wherein the evaluation is executed by an external controller (9) belonging to an extracorporeal monitoring device which, preferably wirelessly, receives measurement data from said sensor (1).
